# EUROPEAN PATENT SPECIFICATION

(11) **EP 1 754 971 B1**
(45) Date of publication and mention of the grant of the patent: **25.06.2008**
(21) Application number: 05747153.4
(22) Date of filing: 29.04.2005
(51) Int. Cl.: G01N 33/50, B65D 81/26, A61J 1/03, B65D 75/36, B65D 75/34

(54) **RAPID DIAGNOSTIC STRIP COMPRISING A MOISTURE-ABSORBING MATERIAL AND THE BLISTER PACKAGING THEREOF**
SCHNELLDIAGNOSESTREIFEN MIT FEUCHTIGKEITSABSORBIERENDEM MATERIAL UND BLISTERPACKUNG DAFÜR
TEST RAPIDE DE DIAGNOSTIC AYANT UNE SUBSTANCE ABSORBANTE DE L'HUMIDITE INTEGREE ET SON EMBALLAGE SOUS FORME DE PLAQUETTE

(30) Priority: 30.04.2004 ES 200401045; 06.05.2004 ES 200401085
(43) Date of publication of application: 21.02.2007
(73) Proprietor: Certest Biotec, S.L., 50018 Zaragoza (ES)
(72) Inventor: LANDETA ELORZ, OSCAR, E-50018 ZARAGOZA (ES); GENZOR ASIN, CARLOS, E-50018 ZARAGOZA (ES)
(74) Representative: PROPI, S.L.
(86) International application number: PCT/ES2005/000235
(87) International publication number: WO 2005/106470

(56) References cited:
- WO-A1-97/32663
- ES-A1- 2 138 890
- ES-T3- 2 101 526
- ES-T3- 2 178 077
- FR-A1- 2 660 634
- US-A- 2 147 384
- US-A- 4 013 566
- US-A- 5 358 118
- US-A- 5 962 333
- US-B1- 6 231 815
- US-B1- 6 319 466

## Description

The present invention relates to a rapid diagnostic test with incorporated moisture absorbent material, which avoids that said moisture may affect the test or even invalidate it, giving erroneous results for the test being performed. Likewise, the invention relates to its form of blister packaging which may be formed in unit form or comprising several units which can be separated from one another.

The object of the invention is, therefore, to provide a new rapid diagnostic test system which incorporates the moisture absorbent material in its own structure, which permits the use of smaller packaging with the consequent reduction in packaging costs, in addition to its smaller transport volume and the smaller surface of moisture permeation from the exterior, this being achieved by suitable blister packaging which permits its properly sealed commercialization.

### BACKGROUND OF THE INVENTION

Currently, there are various products to perform rapid diagnostic tests and new ones are created every day. The best-known ones are immunochromatographic tests, and within these the best known are perhaps rapid pregnancy tests. The great problem with all these types of rapid diagnostic tests is that moisture considerably affects them, changing their characteristics and properties, and may even possibly invalidate them. Therefore, in addition to using packaging, e.g. aluminium or plastics, which guarantee hermeticity to moisture penetration, a type of moisture absorber, such as silica gel, molecular sieve or clay pellets is always added.

There currently exist two clear types of packaging for these products: those which contain multiple tests in a single package, generally for hospital use, aluminium bags being the best-known of this type, wherein a determined number of tests and a pellet or separate sachet with the moisture absorbent material is inserted therein; and, on the other hand, the known plastic tubes wherein a number of tests is also inserted, together with the pellet or sachet of moisture absorbent material. On some occasions, instead of this, packaging manufacturers exist that incorporate the absorbent material in the stopper, which logically increases their price. These types of multiple test packaging have, among others, the great drawback that once the packaging has been opened, it is difficult to guarantee the subsequent shelf-life of the product contained therein.

On the other hand, packages exist which contain a single test, generally intended for personal use and, therefore, with clearly different requirements. The packaging used in these cases is an aluminium bag of reduced dimensions, wherein a test and the sachet or pellet of absorbent material is inserted. This type of single-test packaging has some different types of drawbacks, e.g. a considerably larger bag than the test is necessary so that the sachet or pellet of moisture absorbent material can easily be inserted therein in the filling process, with the consequent increase in packaging costs.

Furthermore, two different operations are required in the filling process, one to insert the test in the packaging and the other to insert the moisture absorbent material, which hinders said packaging process. Another drawback they have is that in many cases the use of these tests is geared towards personal use and not towards qualified personnel, so that there may be confusion in the use of the pellet or sachet of absorbent material instead of the test, with subsequent possible implications, and they may even be ingested.

On the other hand, as the size of the packaging bag is considerably larger than the test, its transport, very important in this area, is considerably affected and its costs are greatly increased.

Finally, the quantity of moisture absorbent material needed is greater as the packaging is larger and, therefore, the moisture permeation surface will also increase.

The blister packaging system as means of packaging and commercialization of products and/or items is very common in different fields and sectors of the art, especially in the pharmaceutical sector, nevertheless, in the diagnostics sector, conventional blister packaging presents serious problems and drawbacks since the tests are seriously affected by moisture, as previously mentioned. In any case, the blister packaging system can be considered as totally rejected in the industrial diagnostic sector, and probably due to the deficiency of traditional aluminiums, wherein micropores exist, as well as the necessary thickness for decent insulation, which involves a large quantity of aluminium necessary to form the blister, also due to the considerable dimensions necessary to blister package the rapid diagnostic test, together with the moisture absorbent material and the high cost of the machinery to manufacture the blister, as well as for the blister packaging process.

ES 2138890 discloses a test device comprises a housing having one or more reactants which may be damaged by humidity during storage wherein the housing is made up, at least partially, of plastic material containing moisture absorbent.

US 6319466 discloses a test device for detecting the presence of a residue analyte in a sample includes a support strip and a sample-absorbing matrix attached to the support strip. The sample-absorbing matrix has a material for absorbing an amount of the sample. The test device also includes a mobile-phase support for holding a mobile-phase composition. The mobile-phase support is attached to the support strip and in contact with the sample-absorbing matrix. A mobile-phase composition is disposed on the mobile-phase support and has a receptor for binding with the analyte. The mobile-phase composition can be carried in the sample. A stationary-phase membrane is attached to the support strip and has a first membrane end in contact with the mobile-phase composition and a second membrane end. The membrane allows lateral capillary flow of the sample from the first membrane end to the second membrane end. A test zone is on the stationary-phase membrane between the first membrane end and second membrane end and having a first binder for binding with an unbound receptor. A control zone is the stationary-phase a membrane between the test zone and second membrane end and has a second binder for binding with an analyte-bound receptor or residual unbound receptor.

### DESCRIPTION OF THE INVENTION

The rapid diagnostic test with incorporated moisture absorbent material and its form of blister packaging which the invention proposes, resolves the aforementioned problem in the different aspects commented in a fully satisfactorily manner.

Thus, the rapid diagnostic test claimed is characterized in that it incorporates a moisture absorbing agent within the test in one of its constituent parts, or in one of the general structural components of said test, which eliminates said moisture during the time in which it remains packaged, so that it does not affect or invalidate it, giving erroneous results when performing the test.

These types of rapid diagnostic tests normally have a plastic base to give them a certain rigidity, or a handling area, so that as the invention proposes, said base or handling area will be the test component that has the best characteristics to incorporate the moisture absorber, since it does not directly interfere with the test, and therefore does not affect its properties as a rapid diagnostic test. Said base or handling area combines one or more polymers with a drying agent, i.e. moisture absorber.

In this way, the base can be formed from a polymer that forms the main part of the composition, e.g. a thermoplastic or thermosetting polymer, a minority nonmiscible polymer with the majority polymer, e.g. a hydrophilic or hydrophobic material and a particle that is substantially found in the minority polymer, e.g. the active agent, i.e. the moisture absorbing agent.

The base with the incorporated absorbent material can be adhered by any method suitable for the test, although it will normally be performed with a double sided adhesive, which means an additional barrier between the test and the absorbent material found in the base.

According to another characteristic of the invention, these rapid diagnostic test with incorporated moisture absorbers are blister packaged. Said blister packaging is based on the blister being formed from two aluminium or plastic sheets, one of them acting as the base wherein one or several cavities is formed for the corresponding test or tests, while the other sheet acts as cover and seal, to close the structures or cavities of the base with the rapid diagnostic tests therein.

The cover and sealing sheet is fixed on the base sheet, by heat fusion, with the mediation of determined polymeric or other materials that belong to the state of the art and which facilitate its fixing throughout its perimeter, to achieve the hermetic sealing of the different cavities formed in the base sheet, in those cases wherein it has more than one structure or cavity.

The blister, either as independent unit or as units which are separable or detachable, by a tear line between two consecutive cavities or structures, will have the surface upon which the sealing or fixing of the cover sheet will be performed, provided with embossing by way of knurling, with the object of permitting better sealing of the cover sheet to the base sheet.

We should highlight the fact that the blister has an external punch-hole adjacent to one of the ends of each separable unit, wherefrom the cover sheet will be separated from the base sheet, which will facilitate the grip and, therefore, the pulling to separate or open the blister of that specific unit.

In a variant of embodiment, an area or space for control code, expiry date, etc. has been provided collateral to each of the units.

Another variant of practical embodiment would be the blister packaging of the test inserted in a plastic case by way of reaction device, which is very widely used in the sanitary product sector for in vitro diagnosis, especially for the rapid diagnostic tests with incorporated moisture absorbers of the invention.

Although the material used would, in principle, be aluminium, the use of other possible materials such as plastics, thermoplastics, etc., especially those with low vapour permeability, cannot be ruled out.

These types of rapid diagnostic tests with incorporated moisture absorber has a series of advantages compared to the methods used up to now to avoid this type of test from being negatively affected by moisture, such as, for example, advantages due to the smaller size of the packaging which makes it easier to transport, only one filling operation is required as the humidity absorber is incorporated in the test, doubt or confusion as to which element or elements are necessary to perform the test is eradicated for individual users.

The blister packaging system for rapid diagnostic test also has several advantages compared to traditional packaging, and among said advantages we can cite the possibility of forming several detachable units in a single body, each with its hermetic test, drastically reducing the dimensions of each unit, as well as the manufacturing costs, given the possibility of making multiple blisters, minimal or practically null moisture transfer, reduction in transport costs and commercialization and permanent maintenance of the test's shelf-life as they are independently sealed.

### DESCRIPTION OF THE DRAWINGS

To complement the description being made and with the object of aiding towards a better understanding of the characteristics of the invention, in accordance with a preferred example of embodiment thereof, a set of drawings is attached as an integral part of said description, wherein the following has been represented, with an illustrative, non-limiting character.
- Figure 1: shows a schematic representation of an immunochromatographic test in the format of a reactive strip where, according to the object of the invention, the moisture absorbent material has been incorporated in the base.
- Figure 2: shows a plan view of a multiple blister of rapid diagnostic tests, object of the invention.
- Figure 3: shows a cutaway view corresponding to the A-A cut line of the previous figure.
- Figure 4: shows another cutaway view, in this case in accordance with the B-B cut line of figure 2.
- Figure 5: shows an alternative embodiment of the multiple blister represented in figure 2, displaying collateral to each structure or cavity of each unit, a rectangular area for control or printing of any type of data or information.

### PREFERRED EMBODIMENT OF THE INVENTION

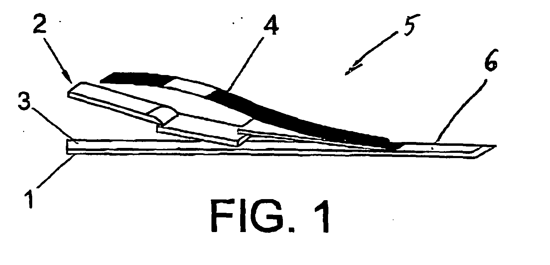

In light of the described figures, and more specifically figure 1, we can observe that the rapid diagnostic test in the form of a reactive strip represented therein is formed from a laminated plastic base (1), which gives it the rigidity necessary for its correct handling, a base which is joined to a structure (2) comprising the test, by means of a double sided adhesive (3), adding one or more protective sheets (4) to the unit, which protect and prevent contamination of the structure (2) or test, thereby avoiding it from being damaged and possible errors from occurring in the test results.

The laminated plastic base (1) is the test element which incorporates the moisture absorbent material, which will be comprised of a combination of one or more polymers together with the drying agent responsible for absorbing the moisture.

As has been previously explained, the test elements (2) can be directly adhered to the base (1) or, as is shown in figure 1, by means of a double sided adhesive (3), which forms an additional barrier between the test and the absorbent material, thus avoiding the test being contaminated.

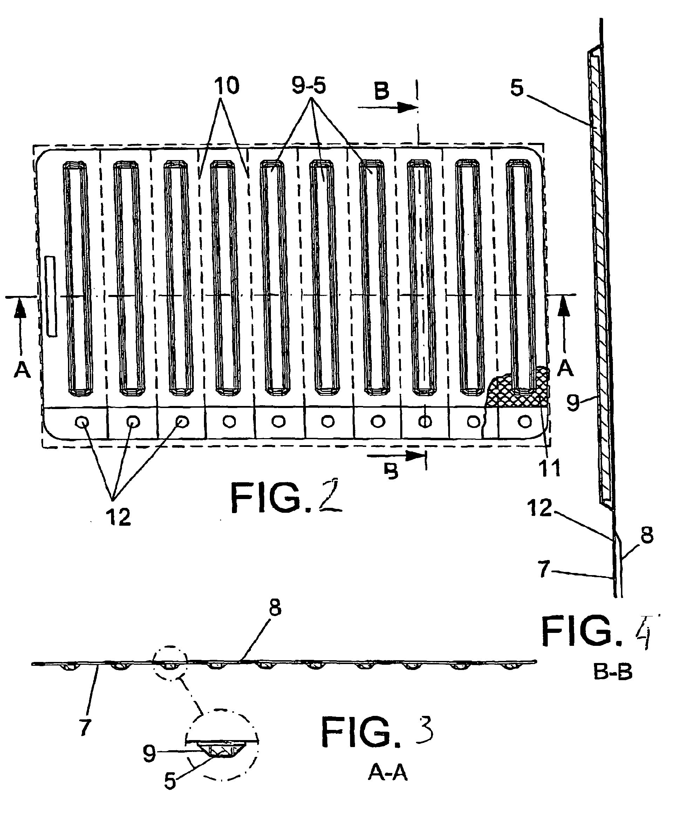

However, a preferred packaging system, represented in figures 2 to 4, is developed in the invention; it is a blister formed from two sheets (7) and (8), the first acting as base sheet and the second as cover and sealing sheet of the previous, so that the base sheet (7) is provided with a series of structures or cavities (9), produced by forming, each one of which is designed to contain a rapid diagnostic test (5).

Each one of these structures or cavities (9) is delimited with respect to the adjacent one, by a pair of weakened or precut lines (10) which permit separating each part or unit determined in the blister.

The cover sheet (8) is fixed to the base sheet (7) by heat fusion, in correspondence with the entire perimeter, to achieve the hermeticity of each of the units determined by the structures (9) with the test (5) therein, since tearing these weakened or precut lines (10) permits separating each unit.

The contact surface of the base sheet (7), whereon the cover sheet (8) is fixed, has embossing (11) by way of knurling, which improves the sealing between both sheets (7) and (8).

Furthermore, adjacent to one of the ends there is a punch-hole (12) wherefrom sheets (7) and (8) are separated, allowing an easy grip and corresponding pulling of the sheet (8) when said cavity corresponding to each blister unit is opened. Evidently, other known opening systems in the blister packaging sector cannot be ruled out.

Having compared the blister packaging disclosed with the traditional packaging system, in both cases using aluminium, it has been verified that the traditional packaging in a bag has an aluminium thickness of approximately 1 micron, whilst the aluminium used in the blister packaging of the invention has an approximate thickness of 20 microns with regard to the cover or sealing sheet (8), and around 45 microns in the base sheet (8), so that thickness will be lost when making the structures in the latter, but it will never be less than the 20 microns of the cover or sealing sheet (8).

Although more material is used in the case of the invention and, therefore, it is more expensive, as a consequence of the drastic reduction in size of one packaging system with respect to the other, it means that the end cost of the blister packaging is practically the same as that of the conventional packaging.

Furthermore, we should highlight the fact that, according to the experiments performed, in traditional packaging the total humidity in the packaging, after one year, is considerably greater than that of the blister packaging of the invention, being able to consider moisture transfer in the blister packaging of the invention as zero.

We should also state that the dimensions of the blister packaging with respect to conventional packaging are considerably reduced in the first case, and we can estimate dimensions of 120 x 60 mm, in conventional packaging, compared to approximately 78 x 5 mm in the blister packaging of the invention, which involves considerable differences between the moisture filtration surface of 0.0144 m² in conventional packaging compared to 0.00078 m² in the blister packaging of the invention, the initial volume of internal air also being considerably lower, which logically has an influence on the initial moisture quantity within the packaging at the time of sealing.

Specifically, it is calculated that the moisture in the conventional packaging, after one year, is 0.377 g compared to 0.008 g in the case of the blister packaging of the invention, which has a clear implication consisting of less need for moisture absorbent material, and therefore greater shelf-life of the product in the blister packaging of the invention.

It should also be highlighted that, as the blister packaging of the invention is of reduced dimensions with respect to conventional packaging, the boxes wherein the products are going to be sent admit between 10 and 34 times more units than with traditional packaging, which means a very large reduction in transport costs, and more so for this type of sector where most of the production is devoted to importation and exportation.

Finally, we should state that even for the case of multi-tests, the blister packaging of the invention has a clear advantage over traditional systems, plastic tubes or bags of greater dimensions, as in these cases once the packaging is open its shelf-life is very difficult to guarantee; in the case of the invention, as the tests are individually hermetically sealed, the shelf-life is not altered.

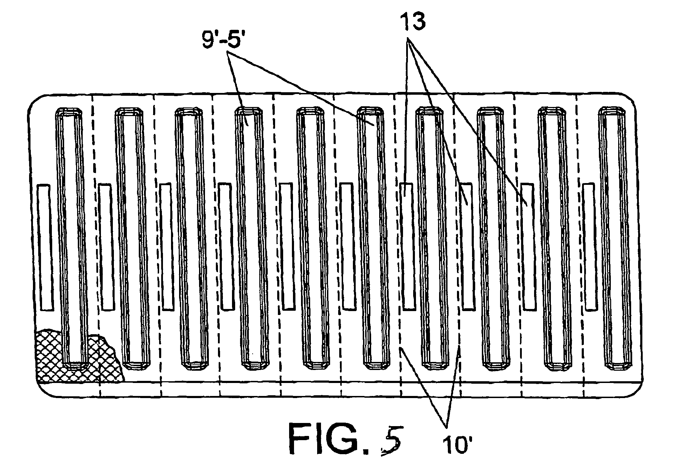

Figure 5 represents an alternative embodiment of the blister also formed from two corresponding sheets, one a base sheet and the other a cover or sealing sheet, with the structures and cavities (9') housing the respective test (5') in each one thereof, so that in this case, each unit, separated as in the previous case from the adjacent ones by delimited tear lines (10'), has, collateral to each cavity or structure (9'), an area for the control code, expiry date information or other type of information, in this case establishing a different opening system, by breaking.

## Claims

1. A blistered rapid diagnostic test **characterized in that** it comprises:
a base (1);
a structure (2);
a moisture absorbing agent integrated in the test (5);
a blister base sheet (7) having at least one cavity (9, 9') so as to define a unit accommodating a rapid diagnostic test (5, 5');
a blister cover sheet (8) which is fixed by heat fusion to the base sheet (7) so as to determine a seal of the at least one cavity (9, 9') containing the rapid diagnostic test (5, 5');

2. The blistered rapid diagnostic test of claim 1 wherein the moisture absorbing agent is integrated with the base (1) in combination with at least one polymer.

3. The blistered rapid diagnostic test of claim 1 wherein the moisture absorbing agent is integrated with a handling area (6) in combination with at least one polymer.

4. The blistered rapid diagnostic test of any of claims 1-3, comprising a punch-hole (12) adjacent to one end of the blister whereby the base sheet (7) and the cover sheet (8) are separated to permit easy gripping and pulling of the cover sheet (8) in an opening operation of the corresponding unit.

5. The blistered rapid diagnostic test of any of claims 1-4, comprising a single cavity (9, 9') determining a single independent unit accommodating a rapid diagnostic test (5, 5').

6. The blistered rapid diagnostic test of any of claims 1-4, comprising a plurality of cavities (9, 9'), each cavity determining a unit accommodating a rapid diagnostic test (5, 5'), said units being independently hermetic and separable via a plurality of weakened tear lines (10, 10') provided between each two cavities (9, 9').

7. The blistered rapid diagnostic teat of any of claims 1-6, further comprising an area where the cover sheet (8) is fixed by heat fusion to the base sheet (7) having a surface with ribs (11) by way of knurling, so as to improve sealing of the cover sheet (8).

8. The blistered rapid diagnostic test of claim 6, further comprising , an area (13) which is adjacent to the weakened tear lines (10') of each unit and collateral to each of the cavities (9'), showing printed data selected from control data, information data and combinations thereof.

9. The blistered rapid diagnostic test of any of claims 1-8, further comprising adhering means for adhering the base (1) to the structure (2) and wherein the base (1) is directly adhered to the structure (2) by the adhering means.

10. The blister rapid diagnostic test of any of claims 1-8, further comprising adhering means for adhering the base (1) to the structure (2), said adhering means comprising a double sided adhesive (3) acting as a separation between the structure (2) and the base (1).

## Patentansprüche

1. Schnelldiagnosetest mit inkorporiertem feuchtigkeitsabsorbierendem Material und seiner Form einer Blisterverpackung, **dadurch gekennzeichnet, dass** er in einem seiner Bestandteile oder in einem seiner Komponenten der allgemeinen Struktur des Test, wie zum Beispiel eine Kunststoffbasis (1) oder der Testausführungsbereich, einen feuchtigkeitsabsorbierenden Wirkstoff inkorporiert, so dass er vollständig in der entsprechenden Struktur integriert ist, in Kombination mit einem oder mehreren Polymeren, wobei vorgesehen ist, dass der besagte Test (5) in einem Blister aus Aluminium oder Kunststoff verpackt ist, der aus einer Grundfolie (7) gebildet ist, mit einer oder mehreren Strukturen, die Vertiefungen (9), (9') oder (9") bilden, die für verschiedene Schnelldiagnosetests (5), (5') oder (5") angeordnet sind, und eine Abdeckung oder Verschlussfolie (8), die durch Fusionswärme mit der Grundfolie (7) verbunden ist, wobei eine Abdeckung jeder der Vertiefungen (9), (9') oder (9") gebildet wird, welche die Tests (5), (5') oder (5") enthalten; mit dem besonderen Merkmal jeder Vertiefung (9), (9') oder (9") mit seinem Test (5), (5') oder (5").

2. Schnelldiagnosetest mit inkorporiertem feuchtigkeitsabsorbierendem Material und seiner Form einer Blisterverpackung nach Anspruch 1, **dadurch gekennzeichnet, dass** die besagte Basis (1) mit dem in seiner Zusammensetzung inkorporieren feuchtigkeitsabsorbierendem Material, direkt auf den Test (2) geklebt wird, durch eine beliebige geeignete Methode oder über einen Doppelklebestreifen (3), der als Trennung zwischen dem Test und dem absorbierenden Material dient.

3. Schnelldiagnosetest mit inkorporiertem feuchtigkeitsabsorbierendem Material und seiner Form einer Blisterverpackung nach Anspruch 1, **dadurch gekennzeichnet, dass** der Blister eine einzelne Struktur oder Vertiefung (9), (9') oder (9") hat, welche eine unabhängige Einheit mit einem entsprechenden Schnelldiagnosetest (5), (5') oder (5") bildet.

4. Schnelldiagnosetest mit inkorporiertem feuchtigkeitsabsorbierendem Material und seiner Form einer Blisterverpackung nach Anspruch 1, **dadurch gekennzeichnet, dass** der Blister mehrere Strukturen oder Vertiefungen (9), (9') oder (9") hat, wobei die gleiche Anzahl an Einheiten mit ihren jeweiligen Schnelldiagnosetests (5), (5') oder (5"), unabhängig hermetisch und über die verdünnten Abreißlinien (10), (10') oder (10 "), welche jeweils zwischen zwei Strukturen oder Vertiefungen vorgesehen sind, voneinander trennbar.

5. Schnelldiagnosetest mit inkorporiertem feuchtigkeitsabsorbierendem Material und seiner Form einer Blisterverpeckung nach einem der vorhergehenden Ansprüche, **dadurch gekennzeichnet, dass** der Bereich oder die Bereiche der Grundfolie (7), auf der die Abdeckung oder die Verschlussfolie (8) durch Fusionswärme befestigt ist, eine Oberfläche mit Rippen (11) hat, über eine Rändelung, die den Verschluss der Verschlussfolie (8) verbessert.

6. Schnelldiagnosetest mit inkorporiertem feuchtigkeitsabsorbierendem Material und seiner Form einer Blisterverpackung nach einem der vorhergehenden Ansprüche, **dadurch gekennzeichnet, dass** es neben einem der Enden jeder Blistereinheit eine Lochung (12) gibt, wobei beide Folien (7) und (8) voneinander getrennte sind, um bei dem Öffnungsvorgang der jeweiligen Einheit ein leichtes Ergreifen und Abziehen der Verschlussfolie (8) zu ermöglichen.

7. Schnelldiagnosetest mit inkorporiertem feuchtigkeitsabsorbierendem Material und seiner Form einer Blisterverpeckung nach Anspruch 1, **dadurch gekennzeichnet, dass** der Blister kollateral zu jeder der Vertiefungen (9') mit deren Test (5') einen Bereich (13) hat, der sich neben den verdünnten Abreißlinien (10') jeder Einheit befindet, wobei der Bereich (13) als Medium für den Aufdruck von Kontrolidaten, Informationen und sonstigen Dingen bestimmt ist.

8. Schnelldiagnosetest mit inkorporiertem feuchtigkeitsabsorbierendem Material und seiner Form einer Blisterverpackung nach Anspruch 1, **dadurch gekennzeichnet, dass** der Blister in Übereinstimmung mit einem der Enden der jeweiligen Vertiefung (9"), welche den entsprechenden Test (5") enthält und die durch Abreißlinien oder verdünnte Linien (10 ") begrenzt sind, einen Bereich (14) für das Pellet (15) oder das feuchtigkeitsabsorbierendem Material hat.

9. Schnelldiagnosetest mit inkorporiertem feuchtigkeitsabsorbierendem Material und seiner Form einer Blisterverpackung nach Anspruch 1, **dadurch gekennzeichnet, dass** das besagte Material optional in einem reaktionsfähigen Streifen (16) inkorporiert ist, der in einem Kunststoffbehälter (17) eingefügt ist.

10. Der geblisterte Schnelldiagnosetest nach jedem der Ansprüche 1-8, der weiterhin Haftmittel zur Anheftung der Grundplatte (1) an der Struktur (2) umfasst, wobei die besagten Haftmittel ein doppelseitiges Klebemittel (3) umfassen, das als eine Trennung zwischen der Struktur (2) und der Grundplatte (1) dient.

## Revendications

1. Test de diagnostic rapide avec matériel absorbeur d'humidité incorporé et sa forme d'emballage coque, **caractérisé en ce qu'** il incorpore dans une de ses parties constituantes ou dans un de ses components de la structure générale du test, comme, par exemple, une base en plastique (1) ou la zone de manipulation du test, un agent absorbeur d'humidité, de façon à ce qu'il soit totalement intégré dans la structure correspondante en combinaison avec un ou plusieurs polymères, étant donné que ledit test est emballé dans une coque en aluminium ou en plastique formée à partir d'une feuille de base (7) avec une ou plusieurs structures de cavités déterminantes (9), (9') ou (9") placées pour différents test de diagnostic rapide (5), (5') ou (5"), et une feuille de couverture ou d'étanchéité (8) fixée par fusion thermique à la feuille de base (7), déterminant un joint pour chacune des cavités (9), (9') ou (9") contenant les tests (5), (5') ou (5"); avec la caractéristique spéciale de chaque cavité (9), (9') ou (9") avec son test (5), (5') ou (5").

2. Test de diagnostic rapide avec matériel absorbeur d'humidité incorporé et sa forme d'emballage coque, selon la revendication 1, **caractérisé en ce que** ladite base (1) avec le matériel absorbeur incorporé dans sa composition est adhérée directement au test (2) par une méthode appropriée ou par moyen d'un adhésif double face (3) qui sert de séparation entre le test et le matériel absorbeur.

3. Test de diagnostic rapide avec matériel absorbeur d'humidité incorporé et sa forme d'emballage coque, selon la revendication 1, **caractérisé en ce que** la coque comprend une seule structure ou cavité (9), (9') ou (9") déterminant une unité indépendante avec son test de diagnostic rapide (5), (5') ou (5") correspondant.

4. Test de diagnostic rapide avec matériel absorbeur d'humidité incorporé et sa forme d'emballage coque, selon la revendication 1, **caractérisé en ce que** la coque comprend plusieurs structures ou cavités (9), (9') ou (9") déterminant la même quantité d'unités avec leurs tests de diagnostic rapide respectifs (5), (5') ou (5"), indépendamment hermétiques et séparables par les lignes de déchirure affaiblies (10), (10') ou (10 ") fournies entre chaque paire de structures ou cavités.

5. Test de diagnostic rapide avec matériel absorbeur d'humidité incorporé et sa forme d'emballage coque, selon les revendications précédentes, **caractérisé en ce que** la zone ou les zones de la feuille de base (7) dans laquelle la feuille de couverture ou d'étanchéité (8) est fixée par fusion thermique comprend une surface à nervures (11) par moletage, qui améliore le scellement de la feuille d'étanchéité (8).

6. Test de diagnostic rapide avec matériel absorbeur d'humidité incorporé et sa forme d'emballage coque, selon les revendications précédentes, **caractérisé en ce que** adjacent à une des extrémités de chaque unité de coque se trouve un trou de poinçon (12) par lequel toutes les deux feuilles (7) et (8) sont séparées pour permettre une préhension et une traction faciles de la feuille de couverture (8) dans l'ouverture de l'unité correspondante.

7. Test de diagnostic rapide avec matériel absorbeur d'humidité incorporé et sa forme d'emballage coque, selon la revendication 1, **caractérisé en ce que** la coque comprend, en position collatérale à chacune des cavités (9') avec son test (5'), une zone (13) qui se trouve adjacente aux lignes de déchirure affaiblies (10') de chaque unité, dont la zone (13) détermine un support pour imprimer les données de commande, information ou autres.

8. Test de diagnostic rapide avec matériel absorbeur d'humidité incorporé et sa forme d'emballage coque, selon la revendication 1, **caractérisé en ce que** la coque comprend, en correspondance avec une des extrémités de la cavité correspondante (9") qui contient les tests respectifs (5") et délimitée par la déchirure ou les lignes affaiblies (10"), une zone (14) pour les pellets (15) ou le matériel absorbeur d'humidité.

9. Test de diagnostic rapide avec matériel absorbeur d'humidité incorporé et sa forme d'emballage coque, selon la revendication 1, **caractérisé en ce que**, facultativement, ledit matériel est incorporé avec une bande réactive (16), insérée dans un boîtier plastique (17).

10. Le test de diagnostic rapide sous blister de n'importe laquelle des revendications 1 à 8, comprenant en outre des moyens adhésifs pour coller la base (1) à la structure (2), lesdits moyens adhésifs comprenant un adhésif double face (3) agissant comme une séparation entre la structure (2) et la base (1).
